# FASCICULE DE BREVET EUROPEEN

(11) **EP 2 324 885 B1**
(45) Date de publication et mention de la délivrance du brevet: **24.10.2018**
(21) Numéro de dépôt: 10176951.1
(22) Date de dépôt: 15.09.2010
(51) Int. Cl.: A61N 1/37, A61N 1/368

(54) **DISPOSITIF MÉDICAL ACTIF COMPRENANT DES MOYENS DE TEST DE CAPTURE PAR ANALYSE DU VECTOGRAMME CARDIAQUE**
AKTIVES MEDIZINISCHES GERÄT MIT MITTELN ZUM EINFANGTESTEN DURCH AUSWERTUNG EINES KARDIALEN VEKTOGRAMMS
ACTIVE MEDICAL DEVICE INCLUDING MEANS FOR CAPTURE TESTING THROUGH CARDIAC VECTOGRAM ANALYSIS

(30) Priorité: 18.11.2009 FR 0958137
(43) Date de publication de la demande: 25.05.2011
(73) Titulaire: Sorin CRM SAS, 92143 Clamart Cedex (FR)
(72) Inventeur: HENRY, Christine, 75014 Paris (FR); MILPIED, Paola, 75014 Paris (FR)
(74) Mandataire: Grünecker Patent- und Rechtsanwälte PartG mbB

(56) Documents cités:
- EP-A1- 2 105 843
- EP-A2- 1 995 685
- US-A1- 2005 137 638
- US-A1- 2006 253 164

## Description

L'invention concerne les dispositifs médicaux implantables actifs tels que définis par la Directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif. La stimulation antibradycardique implique la délivrance contrôlée d'impulsions à l'oreillette et/ou au ventricule (dispositifs de type simple ou double chambre). Dans le cas des thérapies de resynchronisation cardiaque CRT la stimulation doit en outre être appliquée conjointement aux deux ventricules (dispositifs de type multisite).

De façon générale, après la stimulation d'une cavité il est important de recueillir l'onde évoquée, c'est-à-dire l'onde de dépolarisation induite par la stimulation de cette cavité, afin de déterminer si la stimulation a été efficace ou non. Ce test (test de capture) est notamment utilisé pour ajuster l'amplitude et/ou la largeur des impulsions de stimulation, c'est-à-dire l'énergie délivrée au site de stimulation.

Il existe de nombreuses techniques pour effectuer ce test de capture, par exemple celle décrite dans le WO 93/02741 A1 ou US 5 411 533 A (ELA Médical). Dans le dispositif décrit par ce document, le test du seuil d'efficacité de la stimulation ou seuil d'entraînement est effectué à intervalles réguliers, par exemple toutes les six heures, par mise en oeuvre d'un algorithme de test automatique. L'amplitude des impulsions de stimulation est ensuite ajustée sur la base du seuil ainsi mesuré, avec une marge de sécurité additionnelle pour tenir compte notamment des aléas sur la détermination du seuil.

Il arrive en outre, avec les dispositifs connus, que l'algorithme de test soit leurré par certaines situations atypiques, par exemple en cas de fusion, c'est-à-dire d'une stimulation déclenchée de façon concomitante à un événement QRS spontané, au moment du test de capture.

Diverses propositions ont été formulées pour remédier à cette difficulté, notamment par le EP 1 216 722 A1 (ELA Médical), qui propose un moyen de détecter une possible situation de fusion et d'invalider le test en cas de suspicion de fusion. Les EP 1995 685 A2 et US 2006/0253 164 A1 décrivent diverses techniques permettant d'améliorer la spécificité du test de présence/absence de capture. Malgré cela, le suivi clinique des patients montre que les différentes techniques conventionnelles de test de capture restent sensibles à diverses anomalies du rythme survenant de manière erratique, susceptibles de leurrer l'algorithme de test et d'entraîner aussi bien des faux positifs que des faux négatifs. Ces anomalies conduisent à un réajustement erroné de l'énergie de stimulation, soit par excès (ce qui constitue une source de surconsommation et donc de réduction de la durée de vie de l'implant), soit par défaut (avec bien sûr dans ce cas apparition d'un risque pour le patient).

On a par ailleurs cherché, comme dans le EP 1 287 849 A1 (ELA Medical), à opérer le test de capture, et le réajustement éventuel de l'énergie de stimulation, non plus à intervalles réguliers (par exemple toutes les six heures) mais en continu, en vérifiant à chaque cycle si la stimulation a été efficace ou non. Ces techniques d'ajustement cycle à cycle, malgré leur réactivité bien supérieure, sont cependant très sensibles à la survenue d'une fusion, ou d'un cycle atypique isolé tel qu'une détection ventriculaire post-auriculaire, un cycle trop rapide ou une extrasystole, qui risquent d'être interprétés à tort comme des pertes de capture - alors même que le seuil d'entraînement n'a pas naturellement augmenté.

De plus, dans le cas d'un dispositif multisite, il est nécessaire d'exécuter autant de tests de capture qu'il existe de sites, ce qui avec la tendance récente à la multiplication des sites conduit à une augmentation substantielle de la durée du test de capture sur l'ensemble des sites.

L'idée de base réside dans la constatation du fait qu'il est possible d'obtenir des paramètres pertinents de la détection de l'onde évoquée à partir de signaux d'électrogramme endocavitaire (signaux EGM) recueillis concurremment sur deux voies distinctes et provenant d'une même cavité, par exemple d'un ventricule.

Les deux voies EGM différentes sont par exemple celle du signal unipolaire (recueilli entre le boîtier et l'une des électrodes distale ou proximale), et celle du signal bipolaire (recueilli entre l'électrode distale et l'électrode proximale).

De façon caractéristique, l'analyse de ces signaux est une analyse bidimensionnelle à partir de la boucle cardiaque ou vectogramme (VGM), qui est la représentation de l'un des deux signaux par rapport à l'autre dans un espace à deux dimensions. Cet espace est typiquement un espace voie unipolaire (en ordonnée) vs. voie bipolaire (en abscisse), chaque battement ou fraction significative de battement étant alors représenté par son vectogramme dans le plan ainsi défini - en faisant donc abstraction de la dimension temporelle. Le document EP 2 105 843 A1 propose une telle analyse pour discriminer entre tachycardies ventriculaires et tachycardies supraventriculaires. On notera incidemment que l'analyse bidimensionelle ou en deux dimensions (2D) évoquée ici ne doit pas être entendue de manière en elle-même limitative. L'invention peut en effet s'appliquer aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

L'invention propose d'effectuer la détection de l'onde évoquée par analyse du VGM enregistré pendant un cycle cardiaque, tout particulièrement à partir d'une mesure de la ressemblance du VGM enregistré pendant ce cycle à celui enregistré pendant un cycle de référence correspondant à une situation bien définie (capture avérée, absence de capture, fusion).

Plus précisément, l'invention propose un dispositif médical actif du type connu par exemple d'après le EP 1 995 685 A2 précité, comprenant : des moyens de stimulation, aptes à délivrer des impulsions électriques de stimulation destinées à être appliquées à une électrode apte à être implantée dans une cavité cardiaque d'un patient ; des moyens de recueil de l'activité électrique du coeur, comprenant des moyens pour produire au moins deux composantes temporelles distinctes à partir de deux signaux EGM distincts d'électrogramme endocavitaire de ladite cavité ; et des moyens de test de capture sur un cycle stimulé à analyser, aptes à détecter la survenue d'une onde de dépolarisation induite par la stimulation de la cavité.

De façon caractéristique de l'invention, les moyens de test de capture comprennent : des moyens pour déterminer une caractéristique 2D non-temporelle représentative dudit cycle cardiaque à analyser, à partir des variations de l'une desdites composantes temporelles en fonction de l'autre ; et des moyens d'analyse bidimensionnelle, aptes à délivrer au moins un paramètre descripteur de ladite caractéristique 2D, et à déterminer la présence ou la perte d'une capture en fonction de ce(s) paramètre(s) descripteur(s).

Les sous-revendications énoncent des formes de mise en oeuvre avantageuses de l'invention.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 est une vue générale montrant une sonde bipolaire implantée au fond du ventricule.
La Figure 2 illustre les signaux EGM obtenus respectivement sur les voies bipolaire ventriculaire et unipolaire ventriculaire de la configuration de la Figure 1.
La Figure 3 illustre la boucle cardiaque obtenue en combinant les deux signaux de la Figure 2.
La Figure 4 illustre les paramètres de caractérisation d'un vectogramme en un point donné, avec la courbure et le vecteur tangent en ce point.
La Figure 5 représente un électrocardiogramme de surface illustrant au cours de battements successifs diverses situations susceptibles d'être prises en compte par l'algorithme de détection de l'invention.
Les Figures 6 à 11 illustrent, pour les diverses situations consécutives illustrées sur l'électrocardiogramme de la Figure 5, respectivement, à gauche le vectogramme correspondant obtenu, et à droite la position d'un descripteur évalué par l'algorithme de caractérisation par rapport à la frontière de décision entre capture et perte de capture.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply, Paradym, Ovatio, Esprit* ou *Rhapsody* produits et commercialisés par ELA Médical, Montrouge, France (Sorin Group).

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. Comme cela a été indiqué plus haut, la technique d'analyse de l'invention consiste à détecter l'onde évoquée consécutive à la stimulation d'une cavité à partir de signaux d'électrogramme (EGM) recueillis sur deux voies distinctes et analysés en deux dimensions.

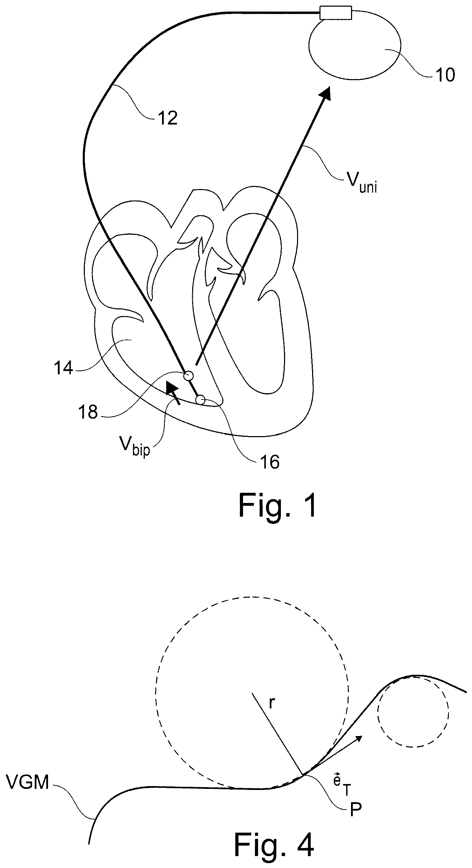

La Figure 1 illustre une configuration de stimulation de type simple chambre dans laquelle un générateur d'impulsions 10 est associé à une sonde 12 implantée dans le ventricule droit 14. La tête de sonde comporte deux électrodes, à savoir une électrode distale 16 et une électrode proximale 18, ce qui permet de recueillir, dans cette configuration la plus simple, un premier électrogramme *Vbip* correspondant à la différence de potentiel mesurée entre l'électrode distale 16 et l'électrode proximale 18, et d'autre part un second électrogramme *Vuni,* mesuré par la différence de potentiel entre l'une des électrodes, par exemple l'électrode proximale 18, et le boîtier métallique du générateur 10.

Cette configuration de type simple chambre a été choisie parce qu'elle est la plus simple pour exposer l'invention, mais elle n'est aucunement limitative. L'invention peut être appliquée de la même façon à la détection de la capture lors d'une stimulation de l'oreillette par une électrode appropriée, ou à la stimulation concomitante des deux ventricules droit et gauche dans le cas des dispositifs multisite, tout particulièrement les dispositifs de stimulation biventriculaire destinés à rétablir la synchronisation entre les deux ventricules. De façon générale, on parlera de cavité, ce terme pouvant s'appliquer indifféremment à l'oreillette ou au ventricule, et aux cavités droites aussi bien qu'aux cavités gauches.

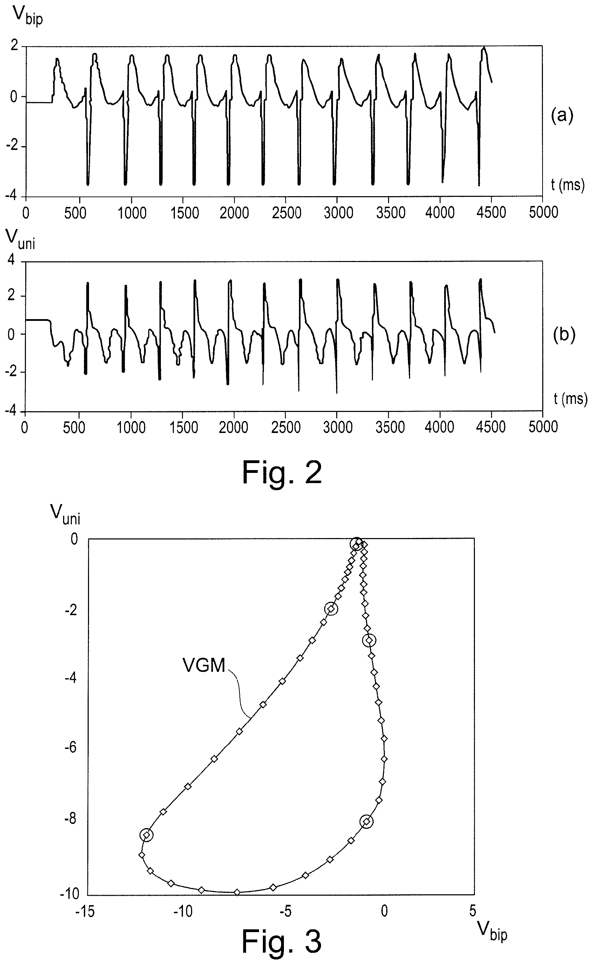

La Figure 2 illustre un exemple de tracés d'électrogrammes *Vbip* et *Vuni* observés respectivement sur la voie bipolaire ventriculaire (Fig. 2a) et sur la voie unipolaire ventriculaire (Fig. 2b) de la configuration de la Figure 1. L'étape suivante consiste, une fois ces signaux recueillis (dans le domaine temporel) à combiner ces signaux et à tracer l'un des signaux EGM en fonction de l'autre.

La Figure 3 illustre la caractéristique correspondante, dénommée "boucle cardiaque" ou "vectogramme" VGM (le vectogramme VGM devant être distingué du vectocardiogramme VCG₁ qui est obtenu à partir de signaux d'électrocardiogramme ECG externes, et non de signaux EGM qui sont des signaux intracardiaques).

Cette boucle VGM constitue donc une représentation d'un battement cardiaque complet dans un espace non temporel.

Il n'est toutefois pas indispensable d'analyser la totalité du battement, l'analyse d'une fraction significative de ce battement (typiquement, celle centrée autour du complexe QRS) étant en général suffisante pour permettre la détection de l'onde évoquée.

Plus précisément, le battement qui suit chaque stimulation est isolé par une fenêtre fixe, par exemple une fenêtre de largeur 100 ms (correspondant à 100 points pour une fréquence d'échantillonnage de 1000 Hz) décalée de 10 ms par rapport à l'instant de la stimulation. Cette valeur typique de 100 ms permet de bien isoler le complexe QRS pour en analyser la morphologie, sans trop inclure de bruit autour, bruit correspondant à la ligne de base après la fin du QRS. Les battements sont enregistrés simultanément sur la voie bipolaire ventriculaire (*Vbip*) et la voie unipolaire ventriculaire (*Vuni*)*.* La fraction de chacun de ces battements comprise à l'intérieur de la fenêtre est alors représentée sous forme de vectogramme, considéré dans le plan constitué de la voie bipolaire en abscisse et de la voie unipolaire en ordonnée. On notera que dans ce cas le vectogramme correspondant n'est pas une boucle fermée, dans la mesure où il ne correspond qu'à une partie de la boucle cardiaque complète, à savoir le complexe QRS isolé à l'intérieur de la fenêtre.

L'invention consiste, essentiellement, à opérer le test de capture, c'est-à-dire la détection de l'onde évoquée, par une analyse du vectogramme, donc une analyse ne faisant pas intervenir le paramètre temporel.

Il s'agit de mesurer le niveau de capture (capture totale, fusion, absence de capture) de la cavité ou des cavités stimulées par le dispositif, notamment :
- pour vérifier que la thérapie a bien été délivrée sur les différents sites stimulés, notamment dans le cas d'une thérapie CRT où il est indispensable que les deux ventricules soient stimulés conjointement ;
- pour vérifier comment la thérapie a été délivrée, à des fins de monitoring (suivi du patient), notamment si l'on veut savoir si, par application de délais de stimulation optimisés sur des caractéristiques hémodynamiques du patient, cette optimisation produit une capture effective ou induit une situation de fusion ;
- pour adapter si nécessaire l'énergie de stimulation au minimum nécessaire, afin de réduire la consommation d'énergie de l'appareil et donc d'en augmenter la durée de vie ;
- pour adapter si besoin les intervalles de stimulation.

La mesure du niveau de capture par le dispositif de l'invention peut se faire cycle à cycle, avec ajustement (ou non) de l'énergie de stimulation ou des intervalles de stimulation (délai atrioventriculaire DAV et/ou délai interventriculaire DVV) en fonction des résultats. Ces réajustements éventuels peuvent aussi bien être réalisés à intervalles réguliers, par exemple toutes les six heures.

On notera incidemment que, dans le cas des dispositifs multisite, le dispositif de l'invention permet, comme on le verra, de tester la présence d'une capture sur tous les sites en même temps, à la différence des techniques actuelles qui imposent, pour s'assurer de la présence d'une capture sur chacun des sites stimulés, de faire des mesures de capture séparément sur chaque site de stimulation programmé.

L'analyse du vectogramme pour le test de capture peut être une analyse intrinsèque des propriétés de la boucle cardiaque, considérée en tant que telle sur le cycle à analyser.

Mais de préférence cette analyse est une analyse comparative, consistant à rechercher une corrélation entre les caractéristiques du vectogramme du cycle à analyser d'une part, et les mêmes caractéristiques relevées sur un ou plusieurs cycles de référence dans des conditions parfaitement déterminées (capture, absence de capture, fusion) d'autre part.

La suite de la description se fera dans ce cadre d'une analyse comparative, mais sans caractère limitatif pour autant.

Dans une forme particulière de mise en oeuvre, qui n'a pas non plus de caractère limitatif, l'invention propose de réaliser la caractérisation du vectogramme par un descripteur basé sur le vecteur tangent *e̅_{T}* en un point P du vectogramme VGM, comme illustré Figure 4.

Il est notamment possible d'utiliser comme descripteur l'angle et/ou la norme de ce vecteur tangent *e̅_{T}*.

Le vecteur tangent *e̅_{T}* en un point donné peut être déterminé par une technique en elle-même connue, notamment avec un filtre discret qui approxime les dérivées premières, par exemple sur quatre points pour une fréquence d'échantillonnage de 1000 Hz.

Un autre descripteur qu'il est possible d'utiliser est la courbure *c* (inverse du rayon de courbure *r* au point P du vectogramme VGM, et ceci pour les différents points échantillonnés successifs de ce vectogramme.

Le vecteur tangent obtenu pour un cycle cardiaque à analyser est comparé au même vecteur pour des courbes de référence qui auront été obtenues au préalable, sur une durée identique, dans différentes conditions de référence telles que notamment :
- capture complète sur tous les sites stimulés, par utilisation d'une stimulation à haute énergie (quand on est donc sûr qu'il y aura toujours capture), ou par confirmation *a posteriori* de la capture par un médecin au vu des cycles enregistrés ;
- capture de certains sites seulement parmi les sites stimulés : une stimulation à haute énergie est délivrée aux sites sur lesquels on veut qu'il y ait capture, et une stimulation à zéro volt sur les autres ;
- perte de capture complète sur tous les sites stimulés, par stimulation à zéro volt de tous les sites.

Tout ce qui ne ressemble pas à ces trois situations sera considéré comme une situation de fusion.

Des courbes de fusion de référence peuvent être créées également en adaptant des intervalles de stimulation par rapport aux événements électriques spontanés présents dans la cavité considérée.

Les vectogrammes de référence sont obtenus soit manuellement, par un test déclenché par le praticien qui valide ensuite chaque type de référence, soit de façon automatique, pour les vectogrammes correspondant à une capture complète et aux pertes partielles ou complètes de capture. Dans ce dernier cas, le dispositif effectue régulièrement (par exemple toutes les quatre heures, toutes les semaines) des tests de stimulation à énergie élevée ou à zéro volt sur les différents sites, ce qui permet de mettre à jour les vectogrammes de référence.

On va maintenant expliquer la manière d'effectuer la comparaison entre le vectogramme du battement analysé et les vectogrammes de référence.

Cette comparaison utilise un critère dérivé d'un ou plusieurs descripteurs permettant d'évaluer le degré de similarité des courbes : aire circonscrite par le vectogramme, angle ou norme du vecteur tangent, sens de parcours, analyse en composantes principales, ou tout autre critère permettant de décrire la morphologie et l'orientation de la courbe dans l'espace utilisé. En fonction du degré de similarité obtenu, le dispositif porte un diagnostic de capture totale, partielle ou nulle, le degré de similarité étant évalué par rapport à des seuils qui peuvent être linéaires ou non.

De préférence, le descripteur est l'angle et/ou la norme du vecteur tangent *e̅_{T}* illustré Figure 4 (voir plus haut).

Dans le cas d'un dispositif biventriculaire par exemple, l'acquisition et la mémorisation préalable des vectogrammes de référence est exécutée par la séquence d'étapes suivante :
- acquisition du vectogramme de référence en capture biventriculaire ;
- délivrance sur huit cycles (par exemple) d'une stimulation biventriculaire à énergie maximale ;
- acquisition des EGM combinés pour chacun de ces huit cycles ;
- moyennage des huit EGM ;
- calcul des critères descriptifs correspondants ;
- stockage des critères descriptifs de la capture biventriculaire complète. Le même procédé est appliqué pour chaque type de vectogramme de référence : capture droite, capture gauche, perte complète de capture à droite et à gauche. Pour être sûr d'obtenir la capture, une énergie maximale est délivrée sur le site considéré ; pour au contraire être certain de ne pas obtenir de capture, la stimulation se fait en énergie nulle sur ce site.

Une fois les électrogrammes de référence acquis et mémorisés, le test de capture sur un cycle cardiaque à analyser est exécuté de la manière suivante :
- stimulation ;
- acquisition du vectogramme sur l'événement courant ;
- calcul des critères descriptifs du vectogramme ainsi acquis ;
- comparaison des critères descriptifs du vectogramme courant par rapport au vectogramme de référence en capture biventriculaire. En cas de différence constatée, la comparaison est effectuée avec les autres vectogrammes de référence (capture droite seule, capture gauche seule), et si une différence est toujours présente on considérera qu'il y a perte de capture - avec dans ce dernier cas délivrance éventuelle d'une contre-stimulation de sécurité à énergie supérieure.

La comparaison des vectogrammes du cycle à analyser avec le ou les vectogrammes de référence est effectuée par des algorithmes tels que ceux décrits dans le EP 2 105 843 A1 (ELA Médical), qui décrit diverses techniques d'analyse comparée d'électrogrammes dans une application spécifique, à savoir la discrimination entre tachycardies ventriculaires et tachycardie supraventriculaires dans un classifieur de tachycardies. Les techniques de comparaison de vectogrammes décrites dans ce document sont toutefois tout à fait transposables à la mise en oeuvre de la présente invention, et l'on pourra se référer à ce document pour plus de détails sur la mise en oeuvre des algorithmes de comparaison.

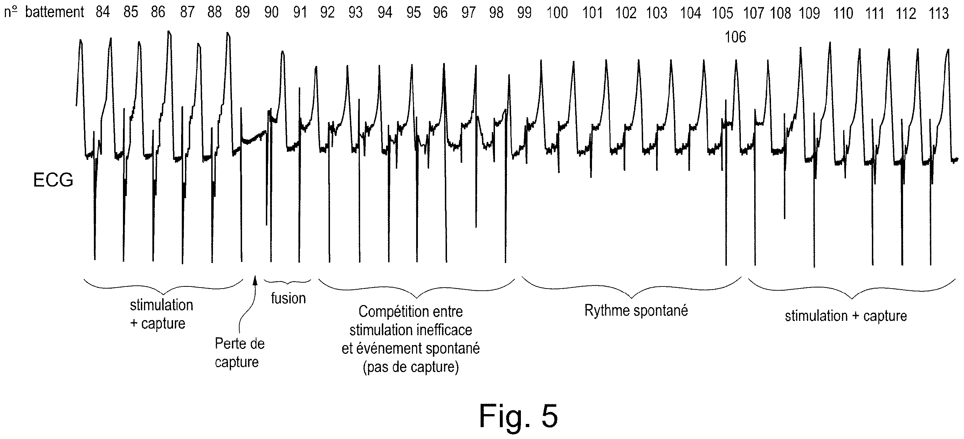

Les Figures 5 à 11 illustrent des exemples de résultats obtenus concrètement par mise en oeuvre d'un test de capture selon l'invention.

La Figure 5 est une représentation d'un électrocardiogramme de surface (ECG) au cours d'un épisode présentant différentes situations telles que capture, perte de capture, fusion, représentatives des situations typiques susceptibles d'être rencontrées en conditions réelles.

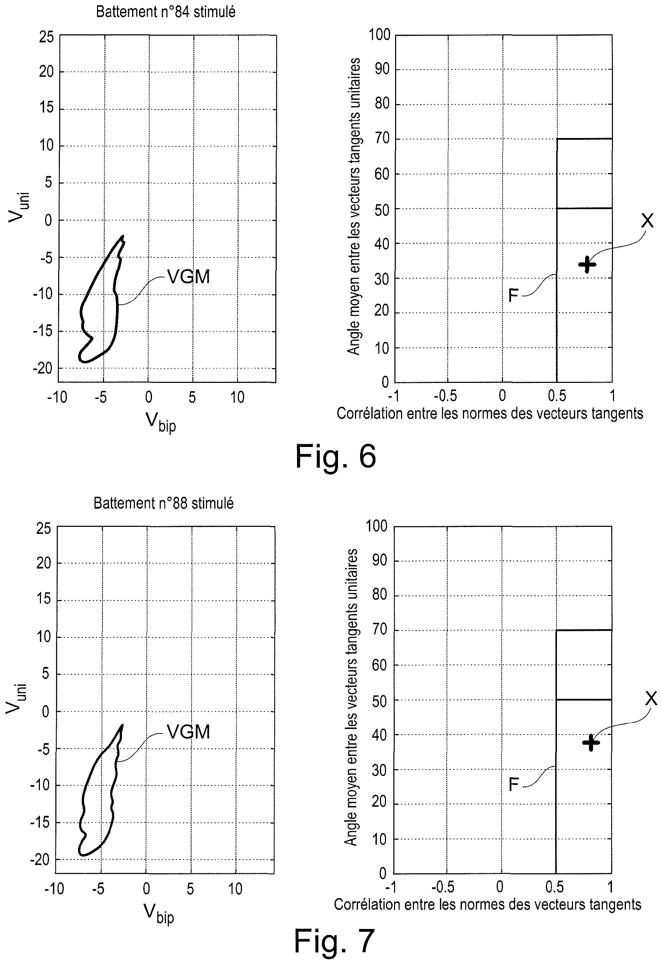

Les Figures 6 à 11 montrent les résultats obtenus avec à gauche le vectogramme correspondant obtenu, et à droite la position d'un descripteur X, évalué par l'algorithme de caractérisation, par rapport à la frontière F de décision entre capture et perte de capture.

Dans cet exemple, le critère retenu est celui d'un double descripteur X combinant (en abscisse) la valeur du coefficient de corrélation entre les normes des vecteurs tangents respectifs du vectogramme analysé et du vectogramme de référence, et d'autre part (en ordonnée) l'angle moyen entre ces mêmes vecteurs tangents respectifs. A partir de ces critères on a défini un domaine correspondant à la frontière F tel que si le double descripteur X se trouve à l'intérieur de ce domaine on considérera qu'il y a capture, et dans le cas contraire qu'il y a perte de capture. Le domaine est par exemple celui représenté par le rectangle correspondant aux critères : coefficient de corrélation > 0,5 et angle moyen < 70°.

Les Figures 6 et 7 correspondent à une situation de capture complète sur battements stimulés (battements n° 84 et 88 de l'ECG de la Figure 5) : on constate que le VGM présente une forme régulière, même juste avant la perte de capture (qui surviendra au battement n° 89). Le double descripteur X est nettement situé à l'intérieur de la frontière de décision F.

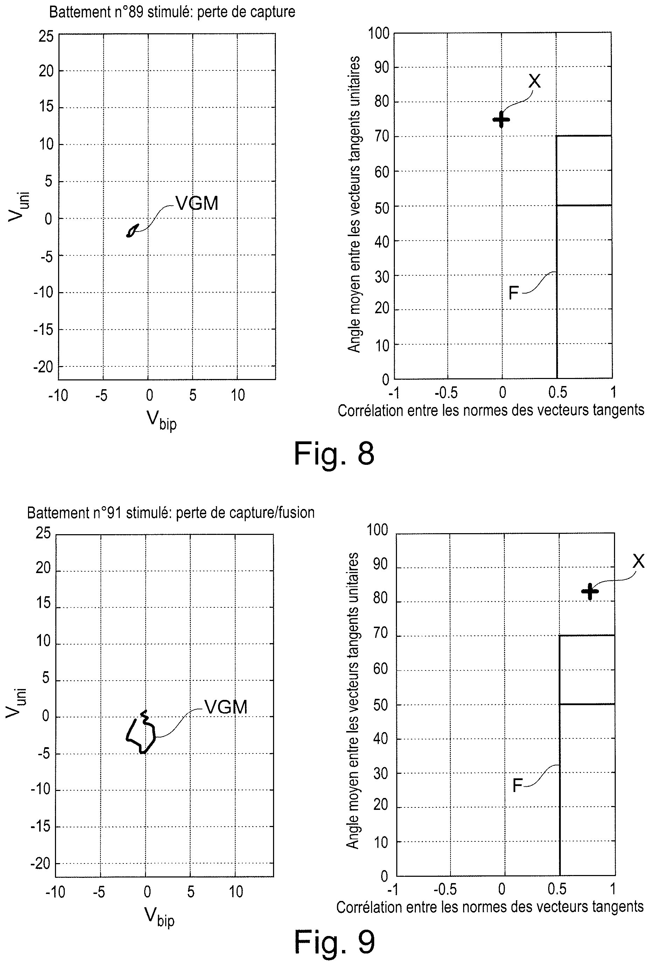

La Figure 8 illustre un battement stimulé avec perte de capture, correspondant au battement n° 89 de l'ECG de la Figure 5. Du fait de l'absence de capture, le VGM se réduit à une boucle de très petites dimensions, dont l'analyse permet toutefois de déterminer un descripteur X situé notablement au-delà de la frontière de décision F, avec notamment un coefficient de corrélation entre les normes des vecteurs tangents qui est voisin de zéro.

La Figure 9 illustre une situation dans laquelle l'absence de capture est liée à l'apparition d'une fusion, correspondant aux battements n° 90 et 91 de l'ECG de la Figure 5. Dans cette situation (Figure 9), on constate que la forme du vectogramme est assez différente de celle d'une stimulation avec capture (Figures 6 et 7) et d'une stimulation sans capture (Figure 8). L'analyse révèle toutefois, malgré un coefficient de corrélation relativement élevé entre les normes des vecteurs tangents, un angle moyen entre ces vecteurs qui dépasse notablement le seuil prescrit. L'algorithme peut dans ce cas indiquer au dispositif que la perte de capture n'est qu'apparente, dans la mesure où elle ne résulte pas d'une augmentation naturelle du seuil d'entraînement, mais est simplement masquée par la survenue d'une fusion.

La distinction entre une perte de capture avérée et une situation de fusion peut être obtenue par exemple par application d'un critère portant sur l'aire circonscrite par le vectogramme, qui sera notablement supérieure dans le cas d'une fusion (Figure 9) que dans le cas d'une véritable perte de capture (Figure 8).

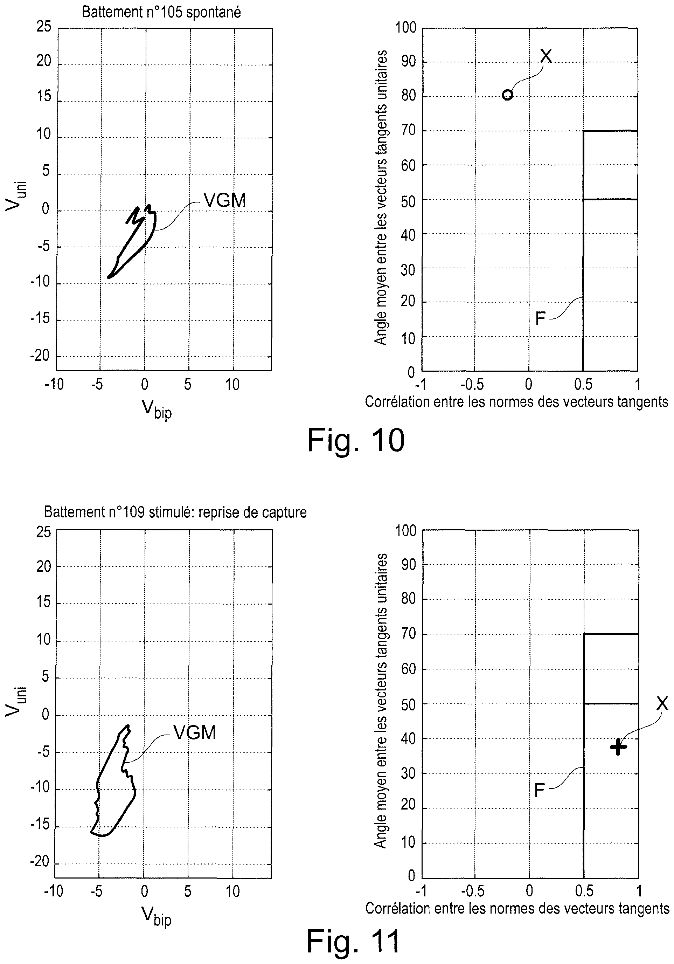

La Figure 10 illustre le cas d'un battement en rythme spontané, correspondant aux battements n° 99 et suivants de l'ECG de la Figure 5. Dans ce cas, l'algorithme inhibe bien évidemment tout test du seuil de capture, qui n'aurait pas de signification.

La Figure 11 illustre enfin la situation après disparition du rythme spontané et reprise de la stimulation et de la capture, correspondant aux battements n° 107 et suivants de l'ECG de la Figure 5 : on retrouve alors des vectogrammes et des descripteurs comparables à ceux des figures 6 et 7. Le test de capture selon l'invention que l'on vient de décrire peut avantageusement être exploité dans le cadre d'une détermination du seuil de stimulation.

À cet effet, le dispositif applique à la cavité des impulsions de stimulation à énergie décroissante, et contrôle à chaque fois la présence ou non d'une onde évoquée par la méthode précédemment décrite. Si à une énergie donnée la capture est avérée, le dispositif considère que la stimulation est une stimulation efficace. L'énergie appliquée pour la stimulation suivante est réduite, typiquement d'un pas d'amplitude fixe, par exemple de 0,25 V. Dès que la capture est perdue, alors le dispositif considère que la stimulation est inefficace, et donc que le seuil de stimulation est supérieur à la dernière valeur appliquée. Dans ce dernier cas, une contre-stimulation de sécurité à amplitude maximale peut être appliquée afin de provoquer en tout état de cause une contraction de la cavité considérée.

Le seuil de stimulation ainsi déterminé peut être conservé dans les mémoires de l'appareil, être transmis à un centre de recueil de données, ou encore utilisé par l'implant pour modifier l'amplitude de la stimulation. Pour d'autres détails sur les algorithmes d'ajustement de l'amplitude de stimulation à partir de tests de capture successifs, on pourra se référer notamment au EP 1 080 744 A1 (ELA Medical), qui décrit diverses techniques de mesure du seuil, de contrôle de cohérence des mesures et d'ajustement de la largeur et de l'amplitude de l'impulsion de stimulation. Les algorithmes correspondants peuvent être mis en oeuvre en utilisant le test de capture effectué par une analyse du vectogramme selon les enseignements de l'invention.

D'autres techniques que celles exposées ci-dessus peuvent être mises en oeuvre pour analyser le vectogramme et déterminer la présence ou non d'une capture.

Une variante peut notamment mettre en oeuvre une analyse en composantes principales (analyse dite ACP) appliquée au vectogramme.

Cette analyse ACP est une technique en elle-même connue, qui permet de déduire l'axe électrique du coeur et fournir ainsi un indicateur de la direction générale que prend l'onde électrique lorsqu'elle se propage dans les ventricules. La voie de plus grande dynamique est celle qui présente la projection la plus grande, la direction correspondante étant dénommée "axe principal" ; cet axe peut être complété par deux autres axes dits axes secondaires, perpendiculaires entre eux et à l'axe principal (dans le cas présent, on n'effectuera l'analyse qu'en deux dimensions, en ne considérant donc qu'un seul des axes secondaires).

L'analyse en composantes principales ACP permet de définir la base orthonormée dans laquelle sera représenté le vectogramme *Vuni* = f(*Vbip*). Si l'on désigne par *S₁* et *S₂* les deux signaux sur les voies respectives *Vbip* et *Vuni* représentant un battement cardiaque, chaque signal est constitué de N points représentés dans la base des électrodes (*Vbip, Vuni*), les coordonnées du *i*^{ième} point étant (*S₁*(*i*)*, S₂*(*i*))*.* Pour l'analyse en composantes principales, on fait l'approximation que ces *N* points forment une ellipse, ce qui permet de calculer les axes de cette ellipse qui forment la base ACP et la longueur de chacun d'eux. Ces deux valeurs permettent d'une part d'identifier la direction principale de l'ellipse (donc la direction d'étalement du vectogramme) et d'autre part de quantifier sa dimension et son aire. On recherchera donc les coordonnées de ces *N* points dans la base ACP (*P₁, P₂*)*,* ce qui nécessitera le calcul de la matrice de passage de la base (*Vbip, Vuni*) à la base (*P₁, P₂*)*.*

L'analyse en composantes principales permet notamment d'extraire les paramètres descripteurs suivants :
- l'axe principal, qui est le vecteur propre de la matrice de covariance associé à la plus grande valeur propre ;
- l'axe secondaire, qui est le vecteur propre de la matrice de covariance associé à la deuxième valeur propre ;
- les dimensions de ces axes, qui sont les deux valeurs propres de la matrice de covariance ;
- les angles que font les deux axes avec l'axe *OX,* à partir des calculs des sinus et des cosinus.

Pour pouvoir extraire à partir de l'ACP des descripteurs de la morphologie des vectogrammes, chaque signal est ensuite projeté sur sa propre base. Ceci permettra d'observer le signal unidimensionnel correspondant (qui sera donc un signal dans le domaine temporel) puis de comparer les formes afin de détecter la présence ou l'absence de l'onde évoquée, ou constater une indétermination du fait d'une situation de fusion.

## Revendications

1. Un dispositif médical actif (10, 12), comprenant :
- des moyens de stimulation cardiaque, aptes à délivrer des impulsions électriques de stimulation de faible énergie destinées à être appliquées à une électrode (16, 18) apte à être implantée dans une cavité cardiaque (14) d'un patient ;
- des moyens de recueil de l'activité électrique du coeur, comprenant des moyens pour produire au moins deux composantes temporelles distinctes (Vbip, Vuni) à partir de deux signaux EGM distincts d'électrogramme endocavitaire de ladite cavité recueillables concurremment ; et
- des moyens de test de capture sur un cycle stimulé à analyser, aptes à détecter la survenue d'une onde de dépolarisation induite par la stimulation de la cavité ;
où
les moyens de test de capture comprennent :
- des moyens pour déterminer une caractéristique 2D non-temporelle (VGM) représentative dudit cycle cardiaque à analyser, à partir des variations de l'une desdites composantes temporelles (Vuni) par rapport à l'autre (Vbip); et
- des moyens d'analyse bidimensionnelle, aptes à délivrer au moins un paramètre descripteur de ladite caractéristique 2D, et à déterminer la présence ou la perte d'une capture en fonction de ce(s) paramètre(s) descripteur(s).

2. Le dispositif de la revendication 1, dans lequel les moyens de recueil sont des moyens aptes à être reliés à deux électrodes (16, 18) d'une sonde placée dans ladite cavité cardiaque, ainsi qu'au boîtier (10) du dispositif, de manière à recueillir un signal bipolaire (Vbip) et un signal monopolaire (Vuni) constituant respectivement lesdits deux signaux EGM distincts.

3. Le dispositif de la revendication 1, dans lequel les moyens pour déterminer une caractéristique 2D consistent en des moyens pour déterminer cette caractéristique à partir des variations desdites composantes temporelles sur une fraction du cycle cardiaque à analyser, dans une fenêtre temporelle incluant le complexe QRS de ce cycle cardiaque.

4. Le dispositif de la revendication 1, dans lequel les moyens d'analyse bidimensionnelle comprennent des moyens pour comparer ladite caractéristique 2D représentative du cycle cardiaque à analyser à au moins une caractéristique 2D de référence obtenue dans des conditions données, le paramètre descripteur étant un paramètre représentatif du degré de similarité des caractéristique 2D courante et de référence.

5. Le dispositif de la revendication 1, dans lequel les moyens d'analyse bidimensionnelle comprennent des moyens également aptes pour la détermination de la survenue d'une situation de fusion en fonction du(des)dit(s) paramètre(s) descripteur(s).

6. Le dispositif de la revendication 1, dans lequel ledit paramètre descripteur délivré par les moyens d'analyse bidimensionnelle est un descripteur géométrique.

7. Le dispositif de la revendication 6, dans lequel le descripteur géométrique est l'angle du vecteur tangent (eT) à la caractéristique 2D, considéré en une pluralité de points (P).

8. Le dispositif de la revendication 7, dans lequel les moyens d'analyse bidimensionnelle comprennent des moyens pour évaluer un coefficient de corrélation (X) entre les angles des vecteurs tangents respectifs de la caractéristique 2D courante et d'une caractéristique 2D de référence.

9. Le dispositif de la revendication 6, dans lequel ledit descripteur géométrique est la norme du vecteur tangent (eT) à la caractéristique 2D, considéré en une pluralité de points (P).

10. Le dispositif de la revendication 9, dans lequel les moyens d'analyse bidimensionnelle comprennent des moyens pour évaluer un coefficient de corrélation (X) entre les normes des vecteurs tangents respectifs de la caractéristique 2D courante et d'une caractéristique 2D de référence.

11. Le dispositif de la revendication 6, dans lequel ledit descripteur géométrique est la courbure (1/r) de la caractéristique 2D, considérée en en une pluralité de points.

12. Le dispositif de la revendication 11, dans lequel les moyens d'analyse bidimensionnelle comprennent des moyens pour évaluer un coefficient de corrélation entre les courbures respectives de la caractéristique 2D courante et d'une caractéristique 2D de référence.

13. Le dispositif de la revendication 6, dans lequel ledit descripteur géométrique est l'aire circonscrite par la caractéristique 2D.

14. Le dispositif de la revendication 1, comprenant des moyens de détermination de repère par analyse en composantes principales, aptes à produire le(s)dit(s) paramètre(s) descripteur(s).

15. Le dispositif de la revendication 1, où le dispositif est un dispositif multisite dans lequel :
- les moyens de stimulation sont aptes à délivrer des impulsions de stimulation destinées à être sélectivement appliquées sur une pluralité de sites de stimulation, ou seulement sur certains de ces sites ;
- les moyens de recueil sont aptes à produire pour chaque site lesdites au moins deux composantes EGM distinctes ; et
- les moyens de test de capture sont aptes à comparer la condition courante à des conditions de référence pour discriminer entre des situations de : présence de capture sur tous les sites stimulés ; présence de capture sur certains seulement des sites stimulés ; et perte de capture sur tous les sites stimulés.

## Patentansprüche

1. Aktives medizinisches Gerät (10, 12), umfassend:
- Mittel zur Herzstimulation, die geeignet sind, elektrische Stimulationsimpulse von geringer Energie zu liefern, die dazu bestimmt sind, an eine Elektrode (16, 18) angelegt zu werden, die geeignet ist, in eine Herzkammer (14) eines Patienten implantiert zu werden;
- Mittel zur Erfassung der elektrischen Aktivität des Herzens, umfassend Mittel zum Erzeugen mindestens zweier unterschiedlicher Zeitkomponenten (Vbip, Vuni) aus zwei unterschiedlichen EGM-Signalen eines endokardischen Elektrogramms der Kammer, die gleichzeitig erfassbar sind; und
- Mittel zum Einfangtesten auf einem zu analysierenden stimulierten Zyklus, die geeignet sind, das Auftreten einer Depolarisationswelle zu erfassen, die durch die Stimulation der Kammer induziert wird;
wobei die Mittel zum Einfangtesten umfassen:
- Mittel zum Bestimmen eines nicht zeitlichen 2D-Merkmals (VGM), das für den zu analysierenden Herzzyklus repräsentativ ist, auf Basis der Variationen einer der Zeitkomponenten (Vuni) in Bezug zur anderen (Vbip); und
- Mittel zur zweidimensionalen Analyse, die geeignet sind, mindestens einen das 2D-Merkmal beschreibenden Parameter zu liefern, und das Vorhandensein oder den Verlust eines Einfangens in Abhängigkeit von diesem(n) beschreibenden Parameter(n) zu bestimmen.

2. Gerät nach Anspruch 1, bei dem die Erfassungsmittel Mittel sind, die geeignet sind, mit zwei Elektroden (16, 18) einer Sonde, die in der Herzkammer angeordnet ist, sowie mit dem Gehäuse (10) des Geräts verbunden zu sein, um ein bipolares Signal (Vbip) und ein monopolares Signal (Vuni) zu erfassen, die jeweils die zwei unterschiedlichen EGM-Signale darstellen.

3. Gerät nach Anspruch 1, bei dem die Mittel zum Bestimmen eines 2D-Merkmals in Mitteln zum Bestimmen dieses Merkmals auf Basis der Variationen der Zeitkomponenten auf einem Bruchteil des zu analysierenden Herzzyklus in einem Zeitfenster, das den QRS-Komplex dieses Herzzyklus einschließt, bestehen.

4. Gerät nach Anspruch 1, bei dem die Mittel zur zweidimensionalen Analyse Mittel umfassen, um das für den zu analysierenden Herzzyklus repräsentative 2D-Merkmal mit mindestens einem Referenz-2D-Merkmal zu vergleichen, das unter gegebenen Bedingungen erhalten wird, wobei der beschreibende Parameter ein Parameter ist, der für den Ähnlichkeitsgrad des aktuellen 2D-Merkmals und des Referenz-2D-Merkmals repräsentativ ist.

5. Gerät nach Anspruch 1, bei dem die Mittel zur zweidimensionalen Analyse Mittel umfassen, die auch für die Bestimmung des Auftretens einer Fusionssituation in Abhängigkeit von dem(n) beschreibenden Parameter(n) geeignet sind.

6. Gerät nach Anspruch 1, bei dem der von den Mitteln zur zweidimensionalen Analyse gelieferte beschreibende Parameter ein geometrischer Deskriptor ist.

7. Gerät nach Anspruch 6, bei dem der geometrische Deskriptor der Winkel des Tangentialvektors (eT) an das 2D-Merkmal, betrachtet in einer Vielzahl von Punkten (P), ist.

8. Gerät nach Anspruch 7, bei dem die Mittel zur zweidimensionalen Analyse Mittel umfassen, um einen Korrelationskoeffizienten (X) zwischen den Winkeln der jeweiligen Tangentialvektoren des aktuellen 2D-Merkmals und eines Referenz-2D-Merkmals zu bewerten.

9. Gerät nach Anspruch 6, bei dem der geometrische Deskriptor die Norm des Tangentialvektors (eT) an das 2D-Merkmal, betrachtet in einer Vielzahl von Punkten (P), ist.

10. Gerät nach Anspruch 9, bei dem die Mittel zur zweidimensionalen Analyse Mittel umfassen, um einen Korrelationskoeffizienten (X) zwischen den Normen der jeweiligen Tangentialvektoren des aktuellen 2D-Merkmals und eines Referenz-2D-Merkmals zu bewerten.

11. Gerät nach Anspruch 6, bei dem der geometrische Deskriptor die Krümmung (1/r) des 2D-Merkmals, betrachtet in einer Vielzahl von Punkten, ist.

12. Gerät nach Anspruch 11, bei dem die Mittel zur zweidimensionalen Analyse Mittel umfassen, um einen Korrelationskoeffizienten zwischen den jeweiligen Krümmungen des aktuellen 2D-Merkmals und eines Referenz-2D-Merkmals zu bewerten.

13. Gerät nach Anspruch 6, bei dem der geometrische Deskriptor der von dem 2D-Merkmal umschriebene Bereich ist.

14. Gerät nach Anspruch 1, umfassend Mittel zur Bestimmung eines Bezugspunktes pro Hauptkomponentenanalyse umfasst, die geeignet sind, den(die) beschreibenden Parameter zu erzeugen.

15. Gerät nach Anspruch 1, wobei das Gerät ein Multisite-Gerät ist, bei dem:
- die Stimulationsmittel geeignet sind, Stimulationsimpulse zu liefern, die dazu bestimmt sind, selektiv an eine Vielzahl von Stimulationsstellen oder nur an gewisse dieser Stellen angelegt zu werden;
- die Erfassungsmittel geeignet sind, für jede der Stellen die mindestens zwei unterschiedlichen EGM-Komponenten zu erzeugen; und
- die Einfangtestmittel geeignet sind, die aktuelle Bedingung mit Referenzbedingungen zu vergleichen, um zwischen folgenden Situationen zu unterscheiden: Vorhandensein eines Einfangens an allen stimulierten Stellen; Vorhandensein eines Einfangens nur an gewissen der stimulierten Stellen; und Einfangverlust an allen stimulierten Stellen.

## Claims

1. An active medical device (10, 12), comprising:
- heart stimulation means, capable of delivering low-energy electric stimulation pulses that are intended to be applied to an electrode (16, 18) suitable for being implanted within a heart chamber (14) of a patient;
- means for receiving the electrical activity of the heart, comprising means for producing at least two distinct temporal components (Vbip, Vuni) on the basis of two distinct EGM signals of an intracardiac electrogram of said chamber that can be received concurrently; and
- capture test means for testing capture over a stimulated cycle to be analysed, which means are capable of detecting the occurrence of a depolarization wave induced by the stimulation of the chamber;
where
the capture test means comprise:
- means for determining an atemporal 2D characteristic (VGM) that is representative of said cardiac cycle to be analysed, on the basis of the variations in one of said temporal components (Vuni) with respect to the other (Vbip); and
- two-dimensional analysis means, capable of delivering at least one descriptive parameter describing said 2D characteristic, and of determining the presence or the loss of capture according to this/these descriptive parameter(s).

2. The device of Claim 1, wherein the receiving means are means that are capable of being linked to two electrodes (16, 18) of a probe placed within said heart chamber, as well as to the housing (10) for the device, so as to receive a bipolar signal (Vbip) and a unipolar signal (Vuni) constituting said two distinct EGM signals, respectively.

3. The device of Claim 1, wherein the means for determining a 2D characteristic consist of means for determining this characteristic on the basis of the variations in said temporal components over a fraction of the cardiac cycle to be analysed, in a time window including the QRS complex of this cardiac cycle.

4. The device of Claim 1, wherein the two-dimensional analysis means comprise means for comparing said 2D characteristic that is representative of the cardiac cycle to be analysed with at least one reference 2D characteristic obtained under given conditions, the descriptive parameter being a parameter that is representative of the degree of similarity of the current and reference 2D characteristics.

5. The device of Claim 1, wherein the two-dimensional analysis means comprise means that are also capable of determining the occurrence of a merging situation according to said one or more descriptive parameters.

6. The device of Claim 1, wherein said descriptive parameter delivered by the two-dimensional analysis means is a geometric descriptor.

7. The device of Claim 6, wherein the geometric descriptor is the angle of the vector that is tangent (eT) to the 2D characteristic, considered at a plurality of points (P).

8. The device of Claim 7, wherein the two-dimensional analysis means comprise means for evaluating a coefficient of correlation (X) between the angles of the respective tangent vectors of the current 2D characteristic and of a reference 2D characteristic.

9. The device of Claim 6, wherein said geometric descriptor is the norm of the vector that is tangent (eT) to the 2D characteristic, considered at a plurality of points (P).

10. The device of Claim 9, wherein the two-dimensional analysis means comprise means for evaluating a coefficient of correlation (X) between the norms of the respective tangent vectors of the current 2D characteristic and of a reference 2D characteristic.

11. The device of Claim 6, wherein said geometric descriptor is the curvature (1/r) of the 2D characteristic, considered at a plurality of points.

12. The device of Claim 11, wherein the two-dimensional analysis means comprise means for evaluating a coefficient of correlation between the respective curvatures of the current 2D characteristic and of a reference 2D characteristic.

13. The device of Claim 6, wherein said geometric descriptor is the area circumscribed by the 2D characteristic.

14. The device of Claim 1, comprising means for determining coordinates by principal component analysis, capable of producing said one or more descriptive parameters.

15. The device of Claim 1, where the device is a multisite device wherein:
- the stimulation means are capable of delivering stimulation pulses that are intended to be selectively applied to a plurality of stimulation sites, or only to some of these sites;
- the receiving means are capable of producing, for each site, said at least two distinct EGM components; and
- the capture test means are capable of comparing the current condition with reference conditions so as to discriminate between situations of: presence of capture across all of the stimulated sites; presence of capture across only some of the stimulated sites; and loss of capture across all of the stimulated sites.
